# FASCICULE DE BREVET EUROPEEN

(11) **EP 4 481 380 B1**
(45) Date de publication et mention de la délivrance du brevet: **05.08.2026**
(21) Numéro de dépôt: 24183309.4
(22) Date de dépôt: 20.06.2024
(51) Int. Cl.: G01N 33/483, G01N 21/64

(54) **ÉLÉMENT D'IMAGERIE, DISPOSITIF D'IMAGERIE, SYSTÈME D'IMAGERIE, PROCÉDÉ D'ANALYSE ET PROCÉDÉ DE FABRICATION ASSOCIÉS**
BILDGEBUNGSELEMENT, BILDGEBUNGSVORRICHTUNG, BILDGEBUNGSSYSTEM, ANALYSEVERFAHREN UND HERSTELLUNGSVERFAHREN DAFÜR
IMAGING ELEMENT, IMAGING DEVICE, IMAGING SYSTEM, ANALYSIS METHOD AND MANUFACTURING METHOD THEREFOR

(30) Priorité: 22.06.2023 FR 2306478
(43) Date de publication de la demande: 25.12.2024
(73) Titulaire: Commissariat à l'Energie Atomique et aux Energies Alternatives, 75015 Paris (FR)
(72) Inventeur: RACINE, Benoit, 38054 GRENOBLE CEDEX 09 (FR); HAON, Olivier, 38054 GRENOBLE CEDEX 09 (FR); LAUGIER, Christelle, 38054 GRENOBLE CEDEX 01 (FR)
(74) Mandataire: Cabinet Camus Lebkiri

(56) Documents cités:
- US-B1- 6 501 846
- TAKASHI TOKUDA ET AL: "Optical and Electric Multifunctional CMOS Image Sensors for On-Chip Biosensing Applications", MATERIALS, vol. 4, no. 1, 29 December 2010 (2010-12-29), pages 84 - 102, XP055347233, DOI: 10.3390/ma4010084

## Description

### DOMAINE TECHNIQUE DE L'INVENTION

Le domaine technique de l'invention est lié à l'analyse d'un échantillon, par exemple un échantillon biologique, par un système d'imagerie.

L'invention concerne un élément d'imagerie destiné à l'analyse d'un échantillon, ainsi qu'un dispositif d'imagerie et un système d'imagerie associés. L'invention concerne en outre un procédé d'analyse de l'échantillon utilisant le dispositif d'imagerie et un procédé de fabrication du dispositif d'imagerie.

L'invention trouve une application dans les domaines de la biologie et de la santé, en particulier dans le domaine des organes sur puces, ou « organ-on-chip » en anglais.

### ARRIERE-PLAN TECHNOLOGIQUE DE L'INVENTION

Les organes sur puce sont des plateformes de culture cellulaire miniaturisées permettant de reproduire la fonction et la structure d'organes humain à une échelle micrométrique et au plus près de leur fonctionnement dans le corps humain. Il est par exemple connu du document « Optical and Electric Multifunctional CMOS Image Sensors for On-Chip Biosensing Applications » de Takashi Tokuda et al. des capteurs d'image CMOS adaptés pour fournir une image optique et une image électrique d'un échantillon biologique (ADN, neurones...).

Les organes sur puces ont l'avantage de se prêter parfaitement à l'intégration de capteurs, car ils sont fabriqués avec des technologies de fabrication similaires.

Ces capteurs sont essentiels pour accéder aux informations concernant les cellules, leurs interactions, leur prolifération ou encore leur réponse à divers stimuli, par exemples à des stimuli chimiques (ou médicamenteux), et, ainsi, permettre de comprendre le fonctionnement des organes et de tester l'effet de médicaments.

Les organes sur puce appliqués à des modèles cellulaires imitant le système nerveux central offrent ainsi la possibilité de mieux comprendre le fonctionnement du cerveau ainsi que certaines maladies neuro-dégénératives du cerveau, telles que la maladie de Parkinson ou la maladie d'Alzheimer, qui sont liées à des aberrations fonctionnelles des réseaux neuronaux. Ces organes sur puce permettent aussi de tester in-vitro l'effet de traitements ciblés sur ces maladies.

Pour ce type d'organes sur puces, des capteurs électrophysiologiques ont été développés pour imager en temps réel l'activité électrique extracellulaire des neurones.

Cette activité extracellulaire est déclenchée par une évolution temporelle de la tension intracellulaire d'un neurone, appelée potentiel d'action, et est associée à des courants ioniques à travers la membrane cellulaire. Typiquement, un potentiel d'action dure entre 1 et 2 millisecondes et, pendant cette durée, le potentiel de la membrane interne passe de -70mV à +30 mV, puis revient à -70 mV.

Un type de capteur électrophysiologique connu permettant d'associer une imagerie à l'échelle d'un neurone (échelle micrométrique) et à l'échelle d'un réseau de neurones (échelle macroscopique) utilise un réseau de transistors dans une configuration appelée EOS (acronyme de « Electrolyte Oxyde Silicium » en anglais).

Dans cette configuration, les neurones sont dans un bain d'électrolyte qui est porté, via une électrode, à un potentiel de référence. En outre, les transistors ne comportent pas d'électrode métallique de grille.

Le document « Electrical imaging of neuronal activity by multi-transistor-array (MTA) recording at 7,8 µm resolution » de Lambacher et al., Applied Physics A., 2004 décrit ainsi une matrice MTA (acronyme de « Multi-Transistor Array » en anglais) de 16384 transistors à effet de champ fabriqués avec une technologie CMOS (acronyme de « complementary metal oxide silicon » en anglais) et recouverts d'une fine couche isolante de dioxyde de titane (TiO₂). La résolution spatiale est de 7,8 µm, et la surface du capteur est de 1 mm².

Le principe d'enregistrement est le suivant : lorsqu'une cellule neuronale, adhérant sur la couche de TiO₂ en vis-à-vis d'un transistor, émet un potentiel d'action, elle produit à la jonction entre la cellule et la couche de TiO₂ un potentiel extracellulaire qui diffère du potentiel de référence du bain d'électrolyte. Ce changement local du champ électrique est couplé capacitivement, à travers la couche isolante de TiO₂, à la grille du transistor et donne lieu à une modulation du courant source-drain calibrée en fonction du potentiel de champ.

Le document « Signal Transmission from individual mammalian nerve cell to field-effect transistor» de Voelker et al., Small Journal, 2, 206-210, 2005 décrit un réseau de transistors EOS amélioré en ce qu'il permet d'enregistrer l'activité électrique extracellulaire avec un meilleur rapport signal à bruit. Les transistors ne comportent pas de couche de TiO₂, et les neurones sont disposés directement sur l'oxyde de grille des transistors.

Toutefois, cet enregistrement est très sensible à la position des cellules neuronales sur les transistors. Il ne suffit pas en effet qu'une cellule neuronale soit positionnée en vis-à-vis d'un transistor pour qu'elle y génère un signal.

En effet, seule une petite partie du transistor définie par l'électrode de grille est effectivement une zone sensible. Cette partie est limitée par la longueur de grille. Une surface non négligeable du capteur ne contribue donc pas à la détection de l'activité électrique.

Il reste donc un besoin d'un système d'imagerie qui soit moins sensible à la position des cellules neuronales permettant ainsi un contact (ou une connexion) plus robuste de ces cellules neuronales sur celui-ci.

Ce besoin existe plus largement pour l'étude d'échantillons comprenant au moins une source apte à générer un signal électrique.

### RESUME DE L'INVENTION

Dans le domaine des capteurs, on appelle « récepteur » l'élément qui est en contact avec l'échantillon et fournit un signal en réponse à un évènement généré par l'échantillon, et on appelle « transducteur » l'élément qui reçoit un signal sous une forme et le transforme en un signal sous une autre forme.

Les inventeurs ont identifié qu'un obstacle pour répondre au problème évoqué précédemment est lié au fait que, dans le capteur de l'état de l'art, le récepteur et le transducteur sont intégrés l'un dans l'autre. Cela implique que la surface de la zone sensible ne peut pas être agrandie dans le but de diminuer la sensibilité au positionnement et d'améliorer la robustesse de contact sans modifier l'architecture du transistor lui-même.

L'invention offre une solution au problème évoqué précédemment en permettant de réaliser un élément d'imagerie dans lequel le récepteur et le transducteur sont deux éléments distincts reliés électriquement, de sorte qu'il est possible d'obtenir facilement, c'est-à-dire indépendamment du transducteur, une surface sensible de plus grande taille que la surface du transistor CMOS de l'état de l'art.

Un premier aspect de l'invention concerne un élément d'imagerie destiné à l'analyse d'un échantillon et comprenant :
- une électrode adaptée à recevoir l'échantillon,
- un élément électroluminescent, disposé en vis-à-vis de l'électrode, et séparé de l'électrode par une couche protectrice isolante,
- une source de courant commandée en tension, configurée pour alimenter l'élément électroluminescent en courant et comprenant une électrode de commande électriquement reliée à l'électrode, de sorte que ledit élément électroluminescent génère une onde lumineuse en réponse à une tension provenant de l'échantillon.

L'élément électroluminescent peut ici être entendu comme un élément qui produit un rayonnement monochromatique ou polychromatique, par exemple dans la bande spectrale 400-800 nm, par conversion d'énergie électrique lorsqu'un courant électrique le traverse. Les diodes électroluminescentes ou LED (acronyme de « Light Emitting Diode » en anglais) et leurs dérivées, principalement les diodes électroluminescentes organiques ou OLED (acronyme de « Organic Light Emitting Diode » en anglais) sont des exemples d'éléments électroluminescents.

Ainsi, le principe d'imagerie est le suivant :
- un échantillon est disposé sur l'électrode,
- une variation du potentiel électrique de l'échantillon modifie le potentiel électrique de l'électrode.
- Ce potentiel électrique est utilisé pour commander la source de courant, qui fournit, en réponse, un courant à travers l'élément électroluminescent. Ce courant génère alors, dans l'élément électroluminescent, une onde lumineuse dont l'amplitude est indicative du potentiel d'action de la cellule neuronale.

Ainsi, l'élément d'imagerie selon le premier aspect de l'invention comporte un récepteur (l'électrode) et un transducteur (l'élément électroluminescent) qui sont deux éléments distincts car séparés par la couche protectrice, situés en vis-à-vis, et reliés par une liaison électrique (via la source de courant).

Cette configuration de l'élément d'imagerie procure une liberté d'agencement de l'électrode par rapport à l'élément électroluminescent, et permet d'adapter la surface du récepteur, et, en tout cas, de l'augmenter par rapport à la surface d'un transistor CMOS d'une matrice MTA, cela sans modifier le transducteur (ici l'élément électroluminescent).

Le dispositif selon le premier aspect de l'invention peut également présenter une ou plusieurs caractéristiques ci-dessous, considérées individuellement ou selon toutes les combinaisons techniquement possibles.

L'élément électroluminescent est disposé entre la couche protectrice et un substrat support transparent, semi-transparent.

L'électrode et la couche de protection sont transparentes ou semi-transparentes.

Le terme « transparent » désigne le fait de présenter un coefficient de transmission optique supérieur à 60% pour au moins une longueur d'onde de la bande spectrale 400-800nm.

Le terme « semi-transparent » désigne le fait de présenter un coefficient de transmission optique supérieur à 60% pour au moins une longueur d'onde de la bande spectrale 400-800nm.

Ainsi, l'onde lumineuse se propage à travers l'électrode transparente ou semi-transparente, en regard de laquelle elle peut être visualisée et/ou mesurée, par exemple par un photodétecteur optique.

La source de courant comprend un transistor ayant :
- une électrode de grille constituant l'électrode de commande de la source de courant ;
- une électrode de source reliée à l'élément électroluminescent ; et
- une électrode de drain reliée à une borne d'alimentation.

Le transistor est avantageusement une technologie simple permettant de convertir une différence de potentiels en un courant.

Lorsque la source de courant comprend un transistor, le transistor est un transistor en couches minces aussi appelé TFT.

Le transistor en couches minces présente plusieurs avantages, parmi lesquels une bonne compatibilité avec les processus de fabrication microélectroniques, un faible coût et une grande flexibilité, ce qui leur permet de piloter différents types d'éléments électroluminescents.

Les dimensions latérales de l'électrode sont supérieures aux dimensions latérales du transistor.

Ainsi, la surface sensible est augmentée par rapport à l'état de l'art, ce qui permet une meilleure efficacité de détection (moins d'évènements sont « manqués » et la réponse fournie par l'élément d'imagerie reflète plus fidèlement la réalité).

Les dimensions latérales de l'électrode sont de préférence comprises entre 4 µm et 20 µm, et par exemple égales à 15 µm.

Ainsi, la taille de la surface sensible correspond à la taille d'une cellule neuronale. L'élément d'imagerie est alors bien adapté, en taille, pour détecter et suivre l'activité électrique extracellulaire d'une cellule neuronale individuelle.

L'élément électroluminescent peut être une diode électroluminescente organique.

Les diodes électroluminescentes organiques ont une structure relativement simple à base d'une superposition de plusieurs couches semi-conductrices organiques entre deux électrodes dont l'une au moins est transparente ou semi-transparente. Le procédé de fabrication est avantageux en termes de coût et de complexité, puisque les couches semi-conductrices et l'électrode peuvent être déposées pleine plaque (c'est-à-dire sur toute la surface du substrat), ce qui évite des étapes de lithographie et de gravure.

Les diodes électroluminescentes organiques ont aussi l'avantage d'être disponibles dans des dimensions latérales micrométriques (par exemple 10 µm et moins), d'être flexibles et facilement connectables à un circuit électronique de pilotage, par exemple un circuit électronique en technologie CMOS. Elles requièrent, enfin, un courant moins élevé que les diodes électroluminescentes inorganiques.

L'élément électroluminescent peut être une diode électroluminescente inorganique, de préférence une micro-diode électroluminescente inorganique.

Comme les OLEDs, les micro-diodes électroluminescentes ou microLEDs (acronyme de « micro-Light Emitting Diodes » en anglais) ont l'avantage de présenter des dimensions micrométriques bien adaptées à la taille des cellules neuronales, par exemple des dimensions comprises entre 15 µm et 20 µm.

Comparativement aux OLEDs, les microLEDs présentent une luminance plus élevée et une durée de vie plus longue, mais elles requièrent un courant de commande plus élevé et sont plus difficiles à fabriquer.

L'élément d'imagerie comprend en outre un circuit électronique de pilotage configuré pour générer une tension de commande de la source de courant, ledit circuit électronique de pilotage reliant électriquement l'électrode de commande de la source de courant et l'électrode.

Par « circuit électronique de pilotage » on entend un circuit qui produit une tension de commande adaptée pour contrôler, via le courant fourni par la source de courant, certaines caractéristiques d'émission de l'élément électroluminescent. Ces caractéristiques sont, par exemple, la dynamique d'intensité lumineuse, la durée d'émission (lié au temps d'observation), etc.

Un deuxième aspect de l'invention concerne un dispositif d'imagerie comprenant une pluralité d'éléments d'imagerie selon le premier aspect de l'invention.

Les éléments d'imagerie sont de préférence agencés de manière à présenter un premier pas de répétition dans une première direction et un deuxième pas de répétition dans une deuxième direction sécante à la première direction.

Le premier pas de répétition est compris entre 4 µm et 30 µm et préférentiellement égal à 10 µm, et le deuxième pas de répétition présente les mêmes caractéristiques de dimension que le premier pas de répétition.

Ainsi, le pas de répétition est égal ou inférieur à la taille d'une cellule neuronale.

Le dispositif d'imagerie comporte alors un réseau régulier, ou matrice, d'éléments d'imagerie.

Ainsi, le dispositif associe une capacité d'imagerie à plusieurs échelles : l'échelle d'un élément d'imagerie, et l'échelle de la matrice.

Les éléments d'imagerie sont de préférence répartis sur une surface supérieure à 1 cm², par exemple 4 cm².

Ainsi, lorsque les éléments d'imagerie sont dimensionnés pour correspondre à la taille d'une cellule neuronale, le dispositif présente des capacités d'imagerie associant une résolution spatiale micrométrique (par exemple 15 µm) et un champ d'analyse centimétrique (par exemple 4 cm²).

De telles capacités permettent d'imager simultanément l'activité d'un grand nombre de cellules neuronales et d'étudier la corrélation entre cette activité et la fonction des réseaux de neurones. Il est, en outre, possible de multiplexer les conditions expérimentales sur un même échantillon. On évite ainsi d'introduire des variations inter-échantillons pouvant biaiser les mesures. Plusieurs médicaments peuvent être ainsi testés et leurs effets comparés de façon fiable, dans l'optique de mieux prédire l'efficacité de futurs traitements de maladies.

Un troisième aspect de l'invention concerne un système d'imagerie d'un échantillon comprenant :
- un dispositif d'imagerie selon le deuxième aspect de l'invention,
- un capteur d'images disposé en regard dudit dispositif d'imagerie, et adapté à former au moins une image de l'onde lumineuse générée par le dispositif d'imagerie sous l'effet de l'échantillon,
- un processeur adapté à traiter l'image formée par le photodétecteur.

Grâce à la transduction électro-optique, la visualisation, l'enregistrement et l'analyse de l'activité électrique sont réalisées par un système optique (capteur d'images et processeur) situé à distance du dispositif d'imagerie et de l'échantillon.

Ce système optique peut être aussi utilisé pour imager les détails morphologiques ainsi que les mouvements de cet échantillon.

L'association de ces deux modes d'imagerie (électrique et morphologique) permet de corréler l'activité électrique d'une population de cellules neuronales à changements morphologiques et organisationnels ou à des mouvements des cellules.

Un quatrième aspect de l'invention concerne un procédé d'analyse d'un échantillon à l'aide du dispositif d'imagerie selon le deuxième aspect de l'invention, comprenant les étapes suivantes :
- Dépôt de l'échantillon sur le dispositif d'imagerie de sorte que l'échantillon soit au contact d'au moins une des électrodes dudit dispositif d'imagerie, chaque électrode en contact appartenant à un élément d'imagerie correspondant dudit dispositif d'imagerie,
- Détection optique de l'activité électrique de l'échantillon, comprenant une étape de collection d'une onde lumineuse générée par l'élément électroluminescent de l'élément d'imagerie correspondant à l'électrode en contact, l'onde lumineuse étant générée en réponse à une tension provenant de l'échantillon.

L'onde lumineuse générée peut être collectée par un capteur d'images, disposé en regard du dispositif d'imagerie, et dans lequel l'étape de collection est suivie d'une étape d'acquisition, par le photodétecteur, d'une image représentative de l'onde lumineuse.

L'étape d'acquisition de l'image représentative de l'onde lumineuse peut être suivie d'une étape de détermination de la tension provenant de l'échantillon à partir de l'image acquise par le capteur d'images.

Un cinquième aspect de l'invention concerne un procédé de fabrication d'un élément d'imagerie destiné à l'analyse d'un échantillon, comprenant les étapes suivantes :
- Fourniture d'un circuit électronique comprenant un plot de connexion et une source de courant commandée en tension, la source de courant comprenant une électrode de commande reliée électriquement au plot de connexion,
- Formation, sur le circuit électronique, d'un élément électroluminescent,
- Formation d'une couche protectrice transparente, ou semi-transparente, et isolante recouvrant l'élément électroluminescent et le plot de connexion,
- Formation, sur la couche protectrice et en vis-à-vis de l'élément électroluminescent, d'une électrode transparente ou semi-transparente adaptée à recevoir l'échantillon,
- Formation, à travers la couche protectrice, d'un via conducteur s'étendant depuis l'électrode transparente ou semi-transparente jusqu'au plot de connexion.

Dans un mode de mise en œuvre préférentiel de ce procédé de fabrication, le via conducteur et électrode transparente ou semi-transparente sont formés d'un même matériau respectivement conducteur et transparent ou conducteur et semi-transparente.

Le procédé de fabrication peut alors comprendre, pour former l'électrode transparente et le via conducteur, les sous-étapes suivantes :
- Formation, par gravure de la couche protectrice, d'une ouverture s'étendant depuis la surface de la couche protectrice jusqu'au plot de connexion reliée à l'électrode de commande de la source de courant,
- Formation simultanée de l'électrode transparente ou semi-transparente et du via conducteur par dépôt, en une seule étape, du matériau conducteur et transparent ou semi-transparente sur la surface de la couche protectrice, en vis-à-vis de l'élément électroluminescent, et sur des parois de l'ouverture.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 représente schématiquement en vue de dessus un mode de réalisation préférentiel d'un dispositif d'imagerie, sous la forme d'une matrice d'éléments d'imagerie,
- La figure 2 représente schématiquement en vue de coupe transversale la matrice d'imagerie de la figure 1 et illustre son principe de fonctionnement,
- La figure 3 représente schématiquement en vue de dessus un élément d'imagerie de la matrice d'imagerie de la figure 1,
- La figure 4 représente schématiquement en vue de coupe transversale selon un plan de coupe AA l'élément d'imagerie de la figure 3,
- La figure 5 représente schématiquement en vue de coupe transversale selon un plan de coupe BB l'élément d'imagerie de la figure 3,
- La figure 6 est un exemple de schéma électrique simplifié de l'élément d'imagerie de la figure 3,
- La figure 7 représente schématiquement en vue de coupe transversale selon un plan de coupe BB une variante de réalisation de l'élément d'imagerie de la figure 3,
- La figure 8 représente schématiquement en vue de coupe transversale selon un plan de coupe AA un élément d'imagerie alternatif à l'élément d'imagerie de la figure 3,
- La figure 9 représente schématiquement en vue de coupe transversale selon un plan de coupe BB l'élément d'imagerie de la figure 8,
- La figure 10 représente schématiquement un système d'imagerie basé sur le dispositif d'imagerie de la figure 1,
- La figure 11 est un schéma synoptique illustrant l'enchaînement des principales étapes d'un procédé d'analyse d'un échantillon,
- Les figures 12A à 12L représentent schématiquement en vue de coupe selon le plan de coupe BB les étapes ou sous-étapes de fabrication de l'élément d'imagerie de la figure 3.

Sauf précision contraire, un même élément apparaissant sur des figures différentes présente une référence unique.

### DESCRIPTION DETAILLEE

Comme mentionné plus haut, la présente invention concerne notamment un dispositif d'imagerie comprenant une pluralité d'éléments d'imagerie pour détecter et visualiser l'activité électrique d'un échantillon.

Dans la suite de la description, on considérera que l'échantillon est un échantillon biologique tel que des cellules neuronales en culture dans un milieu de culture.

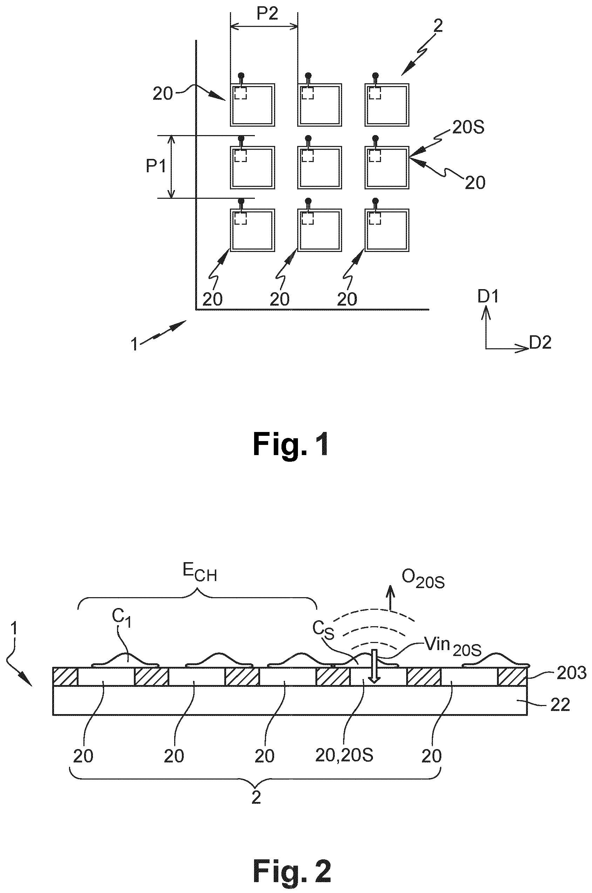

Un mode de réalisation préférentiel de ce dispositif d'imagerie est représenté sur les figures 1 et 2, respectivement selon une vue de dessus et selon une vue en coupe.

Le dispositif d'imagerie 1 comprend *m* x *n* éléments d'imagerie 20. *m* et *n* sont des entiers naturels positifs non-nuls. A titre d'exemple, *m* et *n* peuvent être égaux à 2000.

Les éléments d'imagerie 20 sont du même type, c'est-à-dire fabriqués ensemble et de la même manière, et présentent donc les mêmes caractéristiques ou des caractéristiques très proches. Ils sont de préférence identiques.

Les éléments d'imagerie 20 sont agencés en lignes et en colonnes sous la forme d'une matrice 2. Ils présentent avantageusement un premier pas de répétition P1 dans une première direction D1 et un deuxième pas de répétition P2 dans une deuxième direction D2 sécante à la première direction. La deuxième direction D2 est par exemple perpendiculaire à la première direction D1.

Les pas de répétition P1, P2 peuvent être identiques. Le dispositif d'imagerie comporte alors un réseau régulier, ou matrice, d'éléments d'imagerie.

De préférence, les pas de répétition P1, P2 sont compris entre 4 µm et 30 µm.

De préférence, les pas de répétition P1, P2 sont compris entre 4 µm et 10 µm . Ainsi, les pas de répétition P1, P2 sont égaux ou inférieurs à la taille d'une cellule neuronale.

Le principe général de fonctionnement du dispositif d'imagerie 1 est décrit en relation avec la figure 2.

Le dispositif 1 est destiné à recevoir l'échantillon E_{CH}.

Lorsqu'une des cellules neuronales Cs de cet échantillon E_{CH} émet un potentiel d'action, elle produit un potentiel extracellulaire Vin_{20S} en surface de l'élément d'imagerie 20S avec lequel elle est en contact qui, en réponse, produit localement, en surface du dispositif 1, une onde lumineuse O_{20S} présentant au moins une longueur d'onde, par exemple dans la bande spectrale 400 nm - 800 nm, et dont l'intensité dépend de l'amplitude du potentiel extracellulaire Vin_{20S}.

Supposant que les autres cellules C₁ de l'échantillon E_{CH} ne produisent aucun potentiel d'action (c'est-à-dire qu'elles sont inactives), les autres éléments d'imagerie 20 sont inactifs ou « éteints ».

Comme l'onde lumineuse O_{20S} est localisée au niveau de la cellule électriquement active, et que l'intensité de cette onde lumineuse varie en fonction du potentiel extracellulaire Vin_{20S}, il est possible d'acquérir une cartographie quantitative de l'activité électrique de l'échantillon E_{CH}.

Les éléments d'imagerie 20 sont ainsi comparables à des pixels d'un écran, à la différence que le signal O_{20S} qu'ils délivrent est piloté par, ou fonction de, l'activité électrique Vin_{20S} de l'échantillon Ech.

Le fonctionnement du dispositif d'imagerie 1 sera mieux compris à l'aide de la description de l'élément d'imagerie 20 ci-après.

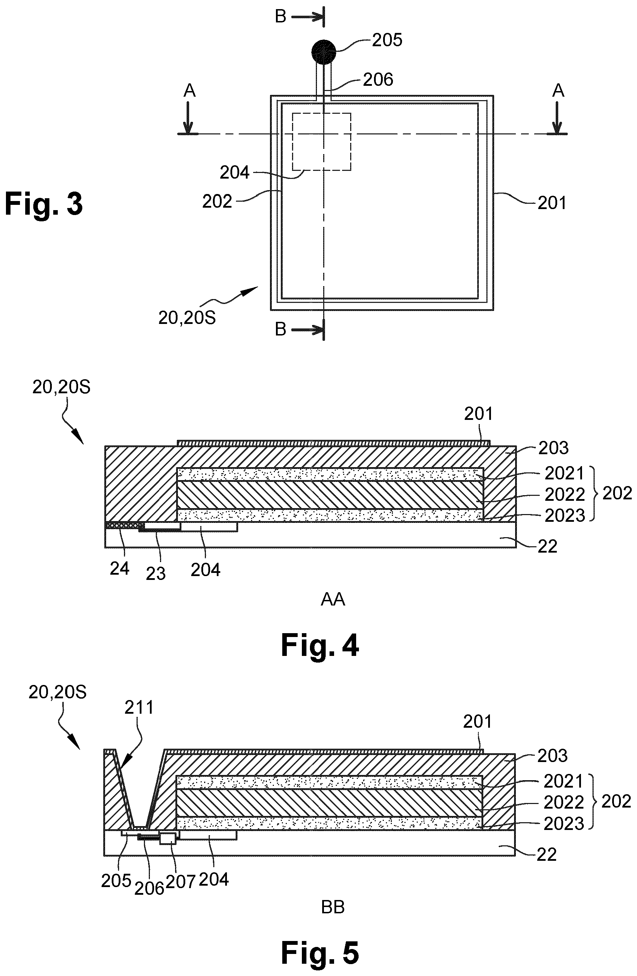

Les figures 3 à 6 représentent schématiquement, selon différentes vues, un premier mode de réalisation de l'élément d'imagerie 20.

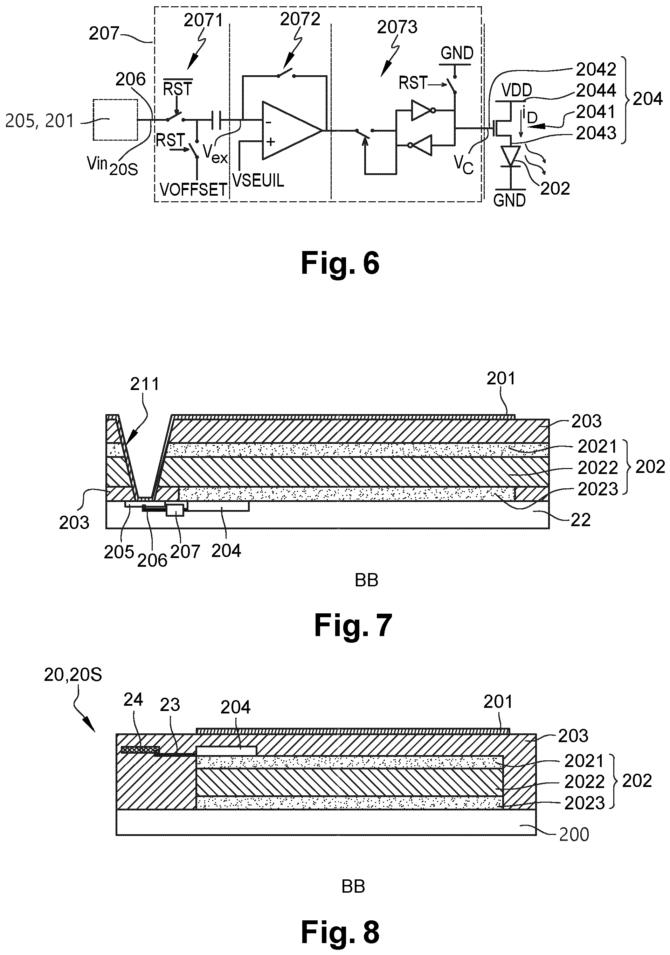

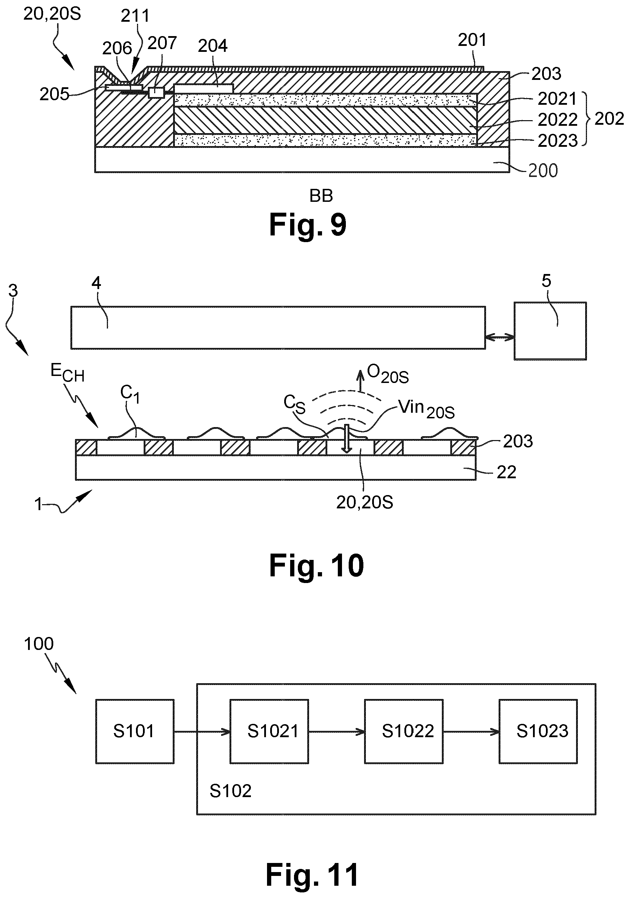

Les figures 8 et 9 représentent schématiquement, selon des vues en coupe, un deuxième mode de réalisation de l'élément d'imagerie 20.

On se référera tout d'abord aux figures 6 et 9 qui représentent les premier et deuxième modes de réalisation de l'élément d'imagerie 20 selon la même vue de coupe BB.

De manière commune à ces deux modes de réalisation, l'élément 20 d'imagerie est destiné à l'analyse de l'échantillon E_{CH} et comprend :
- une électrode 201 adaptée à recevoir l'échantillon E_{CH}
- un élément électroluminescent 202, disposé en vis-à-vis de l'électrode 201, et séparé de l'électrode par une couche protectrice 203 isolante. Par « en vis-à-vis » on entend que tout ou une partie de l'élément électroluminescent 202 se trouve en regard, ou en face, de l'électrode 201.
- une source de courant 204 commandée en tension, configurée pour alimenter l'élément électroluminescent 202 en courant et comprenant une électrode de commande (non représentée sur les figures 6 et 9) électriquement reliée à l'électrode 201.

L'électrode 201 est utilisée comme surface sensible, ou récepteur, c'est-à-dire qu'elle est en contact avec l'échantillon E_{CH}, plus précisément avec une cellule Cs de cet échantillon E_{CH}, et recueille le potentiel extracellulaire Vin_{20S} émis par cette cellule Cs en contact.

Le terme « adaptée à recevoir l'échantillon E_{CH} » signifie que l'électrode n'est pas de nature à dégrader cet échantillon E_{CH} lorsque celui-ci est à son contact. Comme l'échantillon E_{CH} considéré est biologique, l'électrode 201 est biocompatible.

L'électrode 201 présente des dimensions latérales de préférence comprises entre 4 µm et 20 µm, et par exemple égales à 10 µm.

Ces gammes de dimensions sont particulièrement bien adaptées à la taille des cellules neuronales, qui est généralement comprise entre 10 µm ou 15 µm. Plus précisément, ces dimensions sont suffisamment petites pour permettre l'analyse d'une cellule neuronale individuelle, et suffisamment grandes pour que la connexion avec cette cellule individuelle soit robuste par rapport à la position de cette cellule. La surface sensible ainsi obtenue est en effet notamment plus grande que celle décrite précédemment dans l'état de l'art, ce qui permet d'améliorer la capacité de détection d'un potentiel d'action (ou, dit autrement, on diminue le risque de manquer un évènement électrique).

Par ailleurs, comme toute la surface de l'électrode 201 est conductrice, la position de la cellule neuronale sur l'électrode 201 importe peu. Dit autrement, Il n'y a pas nécessité que l'échantillon soit positionné précisément sur une certaine partie de l'élément d'imagerie pour qu'un potentiel d'action soit détecté.

L'élément d'imagerie est ainsi bien adapté à l'imagerie électrique d'une cellule neuronale individuelle, tout en étant, par rapport à l'état de l'art, moins sensible à la position de l'échantillon, ce qui augmente l'efficacité de détection et permet d'obtenir une réponse plus fidèle (plus conforme) à l'activité électrique réelle de l'échantillon.

L'élément électroluminescent 202 est utilisé comme transducteur électro-optique, permettant de produire, comme décrit précédemment en relation avec la figure 2, l'onde lumineuse O_{20S} en réponse au potentiel extracellulaire Vin_{20S} généré par l'échantillon E_{CH} et recueilli par l'électrode 201.

L'élément électroluminescent 202 désigne un élément qui produit un rayonnement non cohérent monochromatique ou polychromatique dans la bande spectrale 400-800 nm, c'est-à-dire dans la bande spectrale de transparence, par conversion d'énergie électrique lorsqu'un courant électrique le traverse.

L'intensité de ce rayonnement est avantageusement proportionnelle au courant électrique.

De préférence, l'élément électroluminescent 202 est une diode électroluminescente organique ou OLED (acronyme de « Organic Light Emitting Diode » en anglais). C'est une technologie qui permet une fabrication et une intégration plus facile et moins coûteuse, et de piloter le dispositif avec des courants de commande moins élevés qu'une LED (acronyme de « Light Emitting Diode » en anglais) par exemple.

La structure interne de l'élément électroluminescent 202 est représentée en particulier en figure 4 et en figure 5 (pour le premier mode de réalisation), et en figures 8 et 9 (pour le deuxième mode de réalisation).

En référence à ces figures 4, 5, 8 et 9, l'élément électroluminescent 202 comprend une couche organique 2022 disposée entre deux électrodes, l'anode 2023 et la cathode 2021 transparentes ou semi-transparentes.

L'élément électroluminescent 202 a des dimensions latérales qui sont, de préférence, proches des dimensions latérales de l'électrode transparente 201. Ainsi, ces dimensions sont de préférence comprises entre 10 µm et 15 µm, par exemple 12 µm.

De telles dimensions permettent d'obtenir une bonne correspondance spatiale entre l'électrode transparente 201 (le récepteur) et l'élément électroluminescent 202 (le transducteur).

Pour cela, en référence aux figures 4 et 5, ou 8 et 9, la cathode 2021, la couche organique 2022 et l'anode 2023 de l'élément électroluminescent 202 sont structurées pour présenter les dimensions latérales souhaitées.

La couche organique 2022 peut être formée de plusieurs couches, par exemple d'une couche HTL (acronyme de « Hole Transporting Layer » en anglais) et d'une couche émissive.

Selon une variante de réalisation représentée par la figure 7, compatible avec les deux modes de réalisation, seule l'anode 2023 de l'élément électroluminescent 202 est structurée pour présenter ces dimensions. Du point de vue de la fabrication, cet agencement simplifie le procédé puisque la couche 2022 organique et la cathode 2021 peuvent être formée par un dépôt en une seule étape, c'est-à-dire sans étape de lithographie et gravure.

L'élément électroluminescent 202 peut aussi être une diode électroluminescente inorganique (ou LED acronyme de « Light Emitting Diode » en anglais), en particulier une microLED.

Comme les OLEDs, les micro-diodes électroluminescentes ou microLEDs (acronyme de « micro-Light Emitting Diodes » en anglais) ont l'avantage de présenter des dimensions micrométriques bien adaptées à la taille des cellules neuronales, par exemple des dimensions comprises entre 15 µm et 20 µm.

Comparativement aux OLEDs, les microLEDs présentent une luminance plus élevée et une durée de vie plus longue, mais elles requièrent un courant de commande plus élevé et sont plus difficiles à fabriquer.

La couche protectrice 203 est une couche d'encapsulation, qui permet de protéger l'élément électroluminescent 202, et, ainsi de garantir sa durée de vie. Elle permet, en outre, d'isoler électriquement l'électrode 201 de l'élément électroluminescent 202.

On remarque que, du fait de l'agencement de la couche protectrice 203 dans l'élément d'imagerie 20, l'électrode 201 est distincte de l'élément électroluminescent 202. En particulier elle est distincte des électrodes, anode 2021 et cathode 2023, de cet élément électroluminescent 202.

La couche protectrice 203 peut aussi entourer l'électrode 201 lorsque celle-ci ne recouvre pas toute la surface de l'élément d'imagerie 20. Elle peut aussi servir à séparer les éléments d'imagerie 20 entre eux dans le dispositif d'imagerie 1 représenté en figure 2. Elle forme alors des blocs isolants séparant les éléments d'imagerie 20. Dans ces deux cas, l'échantillon ECH peut être aussi au contact de la couche protectrice 203 qui est alors, comme l'électrode 201, adaptée à recevoir l'échantillon E_{CH}.

La couche protectrice 203 peut être formée d'un empilement de sous-couches organiques et de sous-couches inorganiques qui peuvent être transparentes.

Les couches inorganiques sont par exemple à base d'oxyde d'aluminium (Al2O3) ou de dioxyde de titane (TiO2). Les couches organiques sont à base de résine.

L'épaisseur de la couche protectrice 203 est de préférence comprise entre 25 nm et 1 µm, et est par exemple égale à 300 nm.

La source de courant 204 permet de piloter l'élément électroluminescent 202 en réponse à l'activité électrique Vin_{20S} de l'échantillon E_{CH}.

Plus précisément, la source de courant 204 a pour effet de convertir le potentiel extracellulaire Vin_{20S} généré par l'échantillon E_{CH} (et recueilli par l'électrode 201) en un courant traversant l'élément électroluminescent 202 et capable de générer l'onde lumineuse O_{20S} dans cet élément électroluminescent 202.

En référence à la figure 5 (premier mode de réalisation) ou à la figure 9 (deuxième mode de réalisation), la connexion électrique entre la source de courant 204 et l'électrode transparente 201 comprend une piste électrique 206, un plot de connexion 205, et un via conducteur 211.

La piste électrique 206 est par exemple métallique.

Le plot de connexion 205 est par exemple métallique.

Le via conducteur 211 s'étend depuis l'électrode transparente 201 jusqu'au plot de connexion 205.

De préférence, le via conducteur 211 est formé du même matériau que l'électrode transparente 201. Le procédé de fabrication s'en trouve facilité car l'électrode 201 et le via 211 sont formés simultanément.

La figure 6 est un exemple d'un schéma électrique simplifié de l'élément d'imagerie 20, compatible avec tous les modes de réalisation de l'élément d'imagerie 20.

En référence à cette figure 6, la source de courant 204 comprend de préférence un transistor 2041 ayant :
- une électrode de grille 2042 constituant l'électrode de commande de la source de courant 204 ;
- une électrode de source 2043 reliée à l'élément électroluminescent 202 ; et
- une électrode de drain 2044 reliée à une borne d'alimentation.

En référence aux figures 4 (premier mode de réalisation) et 8 (deuxième mode de réalisation), cette borne d'alimentation peut être couplée, via des pistes électriques métalliques 23, 24 à une source de tension (non représentée sur la figure 5).

De préférence, le transistor 2041 est un transistor en couches minces aussi appelé **TFT** (acronyme de « Thin Film Transistor » en anglais).

En référence à la figure 6, le transistor 2041 fournit un courant i_{D} entre les électrodes de drain et de source 2043,2044 qui est contrôlée par la tension V_{DD} appliquée à la borne d'alimentation (et donc à l'électrode de drain 2044) et par la tension électrique Vc de l'électrode 2042 de grille. Sur cette figure 6, les références VSEUIL, VOFFSET, RST et RST correspondent à des tensions de commande.

Le transistor 2041 fonctionne en régime saturé.

En l'absence d'activité électrique de la cellule neuronale Cs, le transistor 2041 est bloqué. Aucun courant I_{D} ne circule entre les électrodes de drain 2042 et de source 2043, ni à travers l'élément électroluminescent 202. L'élément électroluminescent 202 est donc éteint et forme un pixel « noir ».

Lorsqu'il y a une activité électrique de la cellule neuronale C_{20S}, le potentiel extracellulaire Vin_{20S} modifie la tension Vc de l'électrode de grille, qui est alors suffisante pour débloquer le transistor 2041. Un courant i_{D}, proportionnel à la tension de grille Vc passe alors à travers l'élément électroluminescent 202 qui émet l'onde lumineuse O_{20S} avec une intensité proportionnelle à la tension de grille Vc.

Dans une phase de calibration, le potentiel électrique sur l'électrode transparente 201 est modulé autour d'un point de fonctionnement et les variations d'intensité de l'onde lumineuse O_{20S} sont mesurées. On obtient ainsi des données de calibration associée à l'élément d'imagerie 20, 20S.

Dans une phase d'analyse, l'intensité lumineuse est mesurée et rapportée, via les données de calibration, à l'amplitude du potentiel extracellulaire V_{in20S}.

En référence à la figure 6, l'élément d'imagerie 20 comprend de préférence un circuit électronique de pilotage 207 relié électriquement, d'une part, à l'électrode de commande 2042 de la source de courant 204, et d'autre part, par l'intermédiaire du plot de connexion 205, à l'électrode transparente 201.

Le circuit de pilotage 207 est configuré pour générer, à partir du potentiel extracellulaire Vi_{n20S}, une tension électrique de commande Vc sur l'électrode de commande 2042 de la source de courant 204.

Pour cela, le circuit 207 de pilotage peut comprendre un ou plusieurs blocs fonctionnels appelés étages, agencés en série.

Par exemple, il comprend un étage interrupteur 2071 permettant de déclencher la lecture de l'électrode transparente 201, et ce faisant, de fournir une tension extracellulaire Vₑₓ à partir du potentiel extracellulaire Vin_{20S} et d'une tension de référence V_{offset}.

Il peut aussi comprendre un étage d'amplification 2072 permettant d'amplifier la tension extracellulaire Vₑₓ de sorte que la tension de commande soit supérieure à la tension de seuil du transistor 2041, cette tension de seuil étant définie comme la tension entre l'électrode de grille 2042 et l'électrode de source 2043 pour laquelle la zone d'inversion apparait.

Ainsi, même des potentiels extracellulaires de faible amplitude, par exemple 15 mV sont détectés et conduisent à l'émission de l'onde lumineuse O_{20S} par l'élément électroluminescent 20).

Il peut aussi comprendre un étage mémoire 2073 permettant d'assurer un maintien sur une certaine durée de la tension électrique Vc sur l'électrode de commande 2042 de la source de courant 204. Cette durée est, au plus, la même que le temps de l'activité neuronale, soit de 1 ms à 2 ms. De préférence, cette durée est adaptée pour garantir un temps d'observation (de l'onde lumineuse) suffisant pour être mesurable, par exemple cette durée est comprise entre 10 µs et 100 µs.

L'étage 2071, l'étage d'amplification 2072 et l'étage mémoire 2073 agencés, dans l'ordre, en série.

L'élément d'imagerie 20 qui vient d'être décrit en relation avec les figures 3 à 9 a plusieurs avantages, notamment :
- Il n'est pas nécessaire de disposer l'échantillon dans un bain d'électrolyte, ni de polariser ce bain d'électrolyte.
- L'élément électroluminescent 202 permet de produire un signal optique représentatif de l'activité électrique neuronale sans qu'il y ait nécessité d'incorporer, dans la cellule, des colorants (par exemple des protéines) sensibles à des variations de la tension cellulaire. L'imagerie est donc non invasive.

Selon le premier mode de réalisation, illustré notamment sur les figures 3, 4 et 5, l'électrode 201 est transparente, ou semi-transparente, de même que la couche protectrice 203.

Le terme « semi- transparent » est dit de tout matériau ou élément qui présente un coefficient de transmission optique supérieur à 60% pour au moins une longueur d'onde comprise dans la bande spectrale s'étendant dans le domaine du visible, soit entre 400 nm et 800 nm.

Le terme « transparent » est dit de tout matériau ou élément qui présente un coefficient de transmission optique supérieur à 80% pour au moins une longueur d'onde comprise dans la bande spectrale s'étendant dans le domaine du visible, soit entre 400 nm et 800 nm.

Ainsi, l'onde lumineuse O_{20S} générée par l'élément électroluminescent 202 en réponse à l'activité électrique de l'échantillon E_{CH} traverse la couche protectrice transparente ou semi-transparente 203 et l'électrode transparente ou semi-transparente 201.

L'onde lumineuse O_{20S} est donc générée au moins en partie du même côté que l'échantillon E_{CH}. Un avantage est qu'il est alors relativement aisé de combiner, à l'aide de systèmes optiques, l'image de l'activité électrique avec l'image des détails morphologiques de l'échantillon E_{CH}.

L'association de ces deux modes d'imagerie (électrique et morphologique) permet de corréler l'activité électrique d'une population de cellules neuronales à changements morphologiques et organisationnels ou à des mouvements des cellules.

L'électrode transparente ou semi-transparente 201 est formée d'un matériau transparent, ou respectivement semi-transparent, et conducteur.

De préférence, ce matériau est choisi parmi les matériaux suivants : le Poly(3,4-ethylenedioxythiophene) (ou PEDOT), l'oxyde d'indium-étain (ou ITO pour « Indium Tin Oxide » en anglais), le dioxyde d'étain (SnO₂), l'oxyde de zinc (ZnO) ou l'oxyde de zinc dopé aluminium (ou AZO).

L'électrode transparente ou semi-transparente 201 peut aussi être formée d'une couche fine, comprise entre 5 nm et 100 nm, d'aluminium (AI), d'argent (Ag), ou de chrome (Cr).

L'électrode 201 est transparente lorsqu'elle présente une épaisseur comprise entre 5 nm et 30 nm.

L'électrode 201 est semi-transparente lorsqu'elle présente une épaisseur comprise entre 5 nm et 100 nm.

Avantageusement, l'anode 2023 de l'élément électroluminescent est une surface de réflexion optique. Ainsi, l'onde lumineuse O_{20S} est générée essentiellement dans la direction de l'électrode transparente ou semi-transparente 201. Cette configuration évite des pertes optiques, et permet d'améliorer la limite de sensibilité de l'élément d'imagerie 20.

En référence à la figure 5, la source de courant 204 commandée en tension fait partie d'un circuit électronique 22. Ce circuit 22 est par exemple couplé à l'élément électroluminescent 202 du côté de l'anode 2023 de cet élément électroluminescent 202. Le circuit électronique 22 sert en outre de substrat, ou de support à l'élément d'imagerie 20, et plus largement au dispositif d'imagerie 1 illustré en figure 1.

La piste électrique 206 est de préférence intégrée au circuit électronique 22.

Le plot de connexion 205 est de préférence intégré au circuit électronique 22. Il est par exemple agencé de sorte à être affleurant à la surface du circuit 22. Il peut également être agencé en saillie (non représenté sur la figure 6).

On remarquera que lorsque le même matériau est utilisé pour former l'électrode 201 et le via conducteur 211, on dispose alors d'un via 211 transparent, ou semi-transparent, permettant d'éviter certains phénomènes optiques (réflexions parasites) pouvant perturber la propagation de l'onde lumineuse O_{20S}.

Selon le deuxième mode de réalisation, illustré sur les figures 8 et 9, l'élément électroluminescent 202 est disposé entre la couche protectrice 203 et un substrat transparent ou semi-transparent 200.

Il en résulte que l'onde lumineuse O_{20S} générée par l'élément électroluminescent 202 en réponse à l'activité électrique de l'échantillon E_{CH} traverse ce substrat transparent ou semi-transparent 200.

Le fait de pouvoir collecter cette onde lumineuse O_{20S} du côté opposé à l'électrode 201 permet de libérer de l'espace au-dessus de l'échantillon E_{CH}, cet espace pouvant être utilisé pour réaliser d'autres types de mesures sur cet échantillon.

Le substrat transparent ou semi-transparent 200 sert de protection et de support à l'élément électroluminescent 202. Il peut s'agir de verre.

L'électrode 201 et la couche protectrice 203 ne sont pas nécessairement transparentes ou semi-transparentes.

Elles peuvent être opaques. Dans ce cas, l'électrode 201 peut être formée d'un métal, par exemple d'aluminium (AI), d'argent (Ag) de chrome (Cr).

Avantageusement, l'électrode 201 est une surface de réflexion. Le métal utilisé pour former cette élctrode 201 peut alors être de l'argnet (Ag). Cela permet d'optimiser la puissance optique traversant le substrat 200, et donc d'améliorer la limite de détection de l'élément d'imagerie 20.

La cathode 2021 de l'élément électroluminescent peut aussi être une surface de réflexion.

En référence à la figure 8, la connexion de la source de courant 204 aux pistes métalliques 23 et 24 est, de manière similaire, agencée de sorte à ne pas bloquer le passage de l'onde lumineuse O_{20S} à travers le substrat 200.

En référence à la figure 9, la source de courant 204, ainsi que les éléments de connexion 205, 206, 211 et le circuit électronique 207 sont agencés de sorte qu'ils ne bloquent pas le passage de l'onde lumineuse O_{20S} à travers le substrat 200. Par exemple, ils peuvent être agencés, au moins en partie, entre l'électrode 201 et l'élément électroluminescent 202.

Quel que soit le mode de réalisation de l'élément d'imagerie 20, l'agencement en niveaux superposés de la source de courant 204, de l'élément électroluminescent 202, de la couche protectrice 203 et de l'électrode transparente 201 permet d'obtenir un élément d'imagerie 20 compact, et intégrant toutes les fonctions de réception/transduction.

Cela permet de réaliser, dans le dispositif d'imagerie 1 représenté sur la figure 1, la matrice 2 d'éléments d'imagerie 20 avec une haute densité, par exemple plus de 6000 électrodes par mm², et de grande surface, par exemple 2x2 cm².

Le dispositif d'imagerie 1 présente ainsi des capacités d'imagerie associant une résolution spatiale micrométrique, compris entre 4 µm et 10 µm, et un champ d'analyse centimétrique, par exemple 4 cm², supérieur à l'état de l'art.

De telles capacités permettent d'imager simultanément l'activité d'un grand nombre de cellules neuronales et d'étudier la corrélation entre cette activité et la fonction des réseaux de neurones. Il est, en outre, possible de multiplexer les conditions expérimentales sur un même échantillon. On évite ainsi d'introduire des variations inter-échantillons pouvant biaiser les mesures. Plusieurs médicaments peuvent être ainsi testés et leurs effets comparés de façon fiable, dans l'optique de mieux prédire l'efficacité de futurs traitements de maladies.

Un autre aspect de l'invention concerne un système 3 d'imagerie, représenté en figure 10, utilisant le dispositif d'imagerie 1.

Ce système 3 permet d'acquérir une image de l'onde lumineuse O_{20S} générée par le dispositif d'imagerie 1 sous l'effet de l'échantillon E_{CH}, puis de réaliser, à partir de cette image, une détection optique de l'activité électrique de l'échantillon E_{CH}.

Le système d'imagerie 3 comprend pour cela le dispositif d'imagerie 1, un capteur d'images 4, et un processeur 5.

Le capteur d'images 4 est formé d'une matrice de pixels et ainsi adapté à former, et acquérir, au moins une image de l'onde lumineuse O_{20S} générée par le dispositif d'imagerie 1 sous l'effet d'un échantillon E_{CH}.

Le capteur d'images 4 est disposé par rapport au dispositif d'imagerie 1 de sorte à collecter l'onde lumineuse O_{20S} générée par un des ou plusieurs des éléments d'imagerie 20S du dispositif d'imagerie 1. Dit autrement, il est disposé du côté de la face du dispositif 1 qui est traversée par l'onde lumineuse O_{20S}.

Sur la figure 10, le dispositif d'imagerie 1 intègre les éléments d'imagerie 20 selon le premier mode de réalisation (dans lesquels l'électrode 201 et la couche protectrice 203 sont transparentes ou semi-transparentes). Ainsi, le photodétecteur 4 est disposé du côté des électrodes 201 de ces éléments d'imagerie 20, et donc du côté de l'échantillon E_{CH}. Naturellement, lorsque les éléments d'imagerie sont réalisés selon le deuxième mode de réalisation, le photodétecteur 4 est disposé du côté opposé aux électrodes 201 et à l'échantillon E_{CH}.

Ce capteur d'image 4 peut être couplé avec la face du dispositif d'imagerie traversée par l'onde lumineuse O_{20S} par une optique de focalisation, telle que lentilles optiques, objectifs, etc.

Le capteur d'images 4 peut être intégré dans un microscope.

Le processeur 5 communique avec le capteur d'images 4. Il s'agit par exemple d'un microprocesseur. Il permet de traiter l'image formée par le capteur d'images, c'est-à-dire de réaliser des opérations de détermination de caractéristiques de l'image, etc.

Un autre aspect de l'invention concerne un procédé d'analyse de l'échantillon E_{CH} à l'aide du dispositif d'imagerie 1.

Ce procédé d'analyse 100 est décrit en relation avec la figure 11. Il comprend les étapes principales S101, S102 et S103.

Le procédé d'analyse 100 débute avec l'étape S101 consistant à déposer l'échantillon E_{CH} sur le dispositif d'imagerie 1, au contact d'au moins une des électrodes 201 de ce dispositif d'imagerie 1. Chaque électrode 201 en contact avec l'échantillon appartient à un élément d'imagerie 20 correspondant dudit dispositif d'imagerie 1, et est associée à l'élément électroluminescent dudit élément d'imagerie 20.

Le procédé 100 d'analyse se poursuit avec l'étape S102, qui consiste à réaliser une détection optique de l'activité électrique de l'échantillon E_{CH}.

Cette détection optique comprend une étape S1021 de collection d'une onde lumineuse O_{20S} générée par l'élément électroluminescent (202) de l'élément d'imagerie correspondant à l'électrode en contact, cette onde lumineuse O_{20S} étant formée en réponse à une tension Vin_{20S} provenant de l'échantillon E_{CH}.

La détection optique peut être réalisée à l'aide du capteur d'images 4 du système d'imagerie 3 (cf. figure 10).

Dans ce cas, la collection S1021 de l'onde lumineuse O_{20S} est réalisée par le capteur d'images 4. L'étape S1021 de collection peut alors être suivie d'une étape d'acquisition S1022, par le capteur d'images 4, d'une image représentative de l'onde lumineuse O_{20S} formée sur le capteur d'images 4.

Cette étape S1022 d'acquisition peut alors se poursuivre par une étape de détermination S1023 de l'activité électrique de l'échantillon, précisément de la tension V_{in20S} provenant de l'échantillon E_{CH} à partir de l'image acquise par le capteur d'images 4.

Cette étape S1023 de détermination est réalisée par le processeur 5 du système d'imagerie 3 (cf. figure 10). Elle peut comprendre :
- une étape de détermination des caractéristiques de l'image acquise, telle que la détermination d'une région d'intérêt correspondant aux éléments d'imagerie actifs (c'est-à-dire lumineux), et la détermination d'une valeur moyenne d'intensité (de niveaux de gris) sur cette région d'intérêt, et
- une étape de corrélation desdites caractéristiques déterminées avec le potentiel extracellulaire généré par l'échantillon ECH, à l'aide des caractéristiques de calibration. Ces caractéristiques de calibration sont établies en mettant en œuvre l'étape de détection optique S102, à l'aide d'un échantillon « étalon » consistant à appliquer un potentiel électrique connu modulé autour d'un point de fonctionnement sur l'électrode transparente 201.

Un autre aspect de l'invention concerne un procédé de fabrication de l'élément 20 d'imagerie.

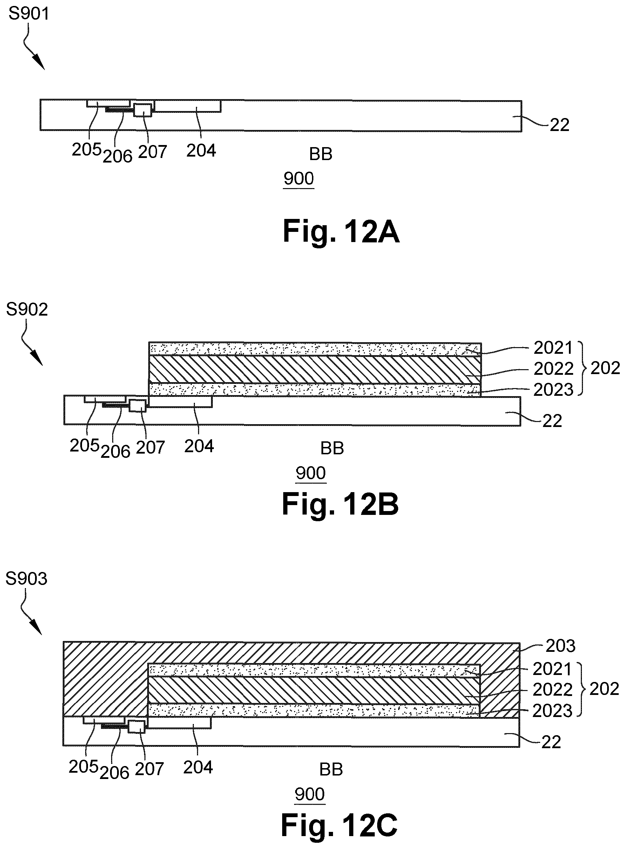

Les figures 12A à 12L illustrent les étapes et sous-étapes de ce procédé 900 de fabrication pour fabriquer l'élément d'imagerie 20 selon le premier mode de réalisation (illustré en figures 3, 4 et 5). On considérera que ce premier mode de réalisation comprend que la couche protectrice 203 et l'électrode 201 sont transparentes.

De façon générale, le procédé 900 comprend les étapes suivantes :
- Fourniture du circuit électronique 22 comprenant le plot de connexion 205 et la source de courant 204 commandée en tension, la source de courant 204 comprenant une électrode de commande (non représentée) reliée électriquement au plot de connexion 205,
- Formation, sur le circuit électronique 22, de l'élément électroluminescent 203,
- Formation de la couche protectrice 203 transparente et isolante recouvrant l'élément électroluminescent 202 et le plot de connexion 205,
- Formation, sur la couche protectrice 203 et en vis-à-vis de l'élément électroluminescent 202, de l'électrode transparente 201 adaptée à recevoir l'échantillon,
- Formation, à travers la couche protectrice 203, du via conducteur 211 s'étendant depuis l'électrode transparente 201 jusqu'au plot de connexion 205 reliée à l'électrode de commande de la source de courant 204.

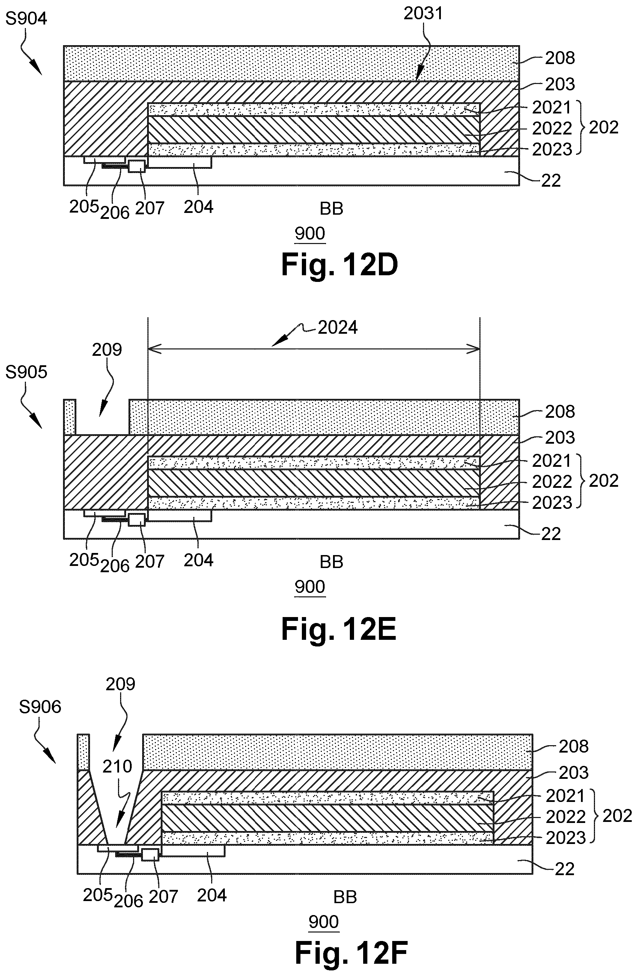

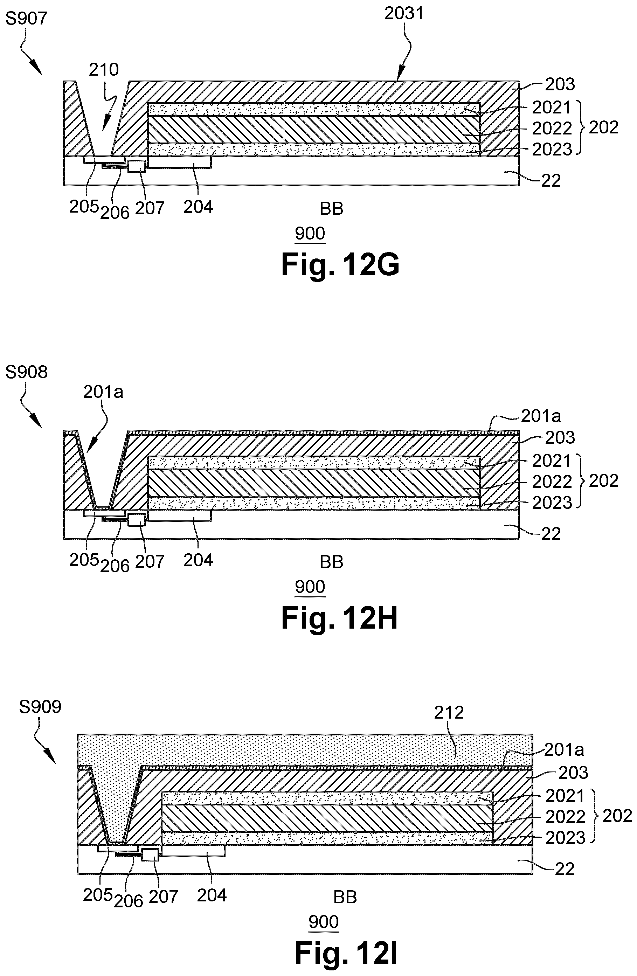

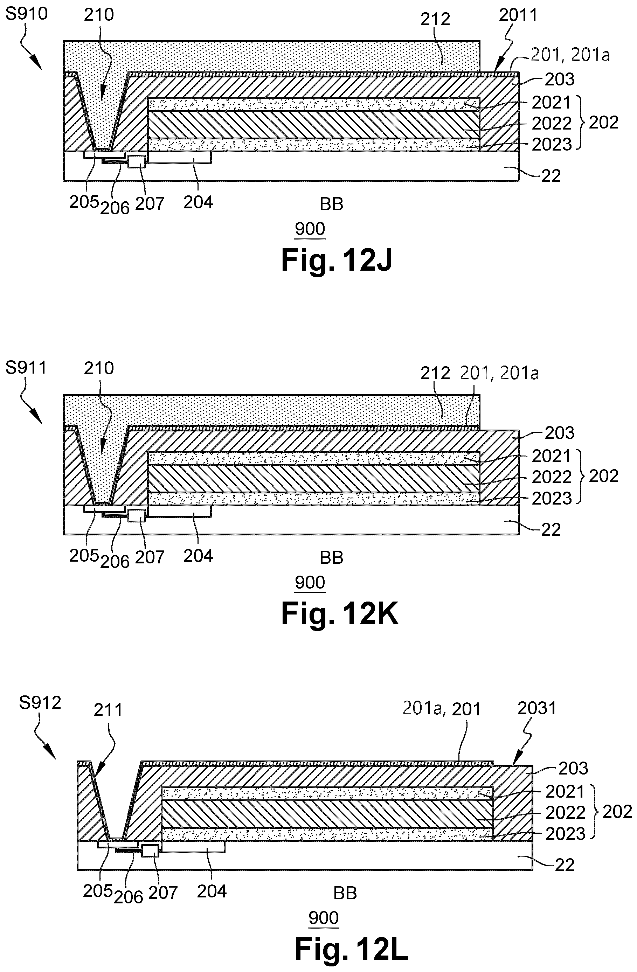

Dans un mode particulier de mise en œuvre, le procédé 900 comprend les étapes S901, S902, S903, S904, S905, S906, S907, S908, S909, S910, S911 et S912 illustrées respectivement en figure 12A, en figure 12B, en figure 12C, en figure 12D, en figure 12E, en figure 12F, en figure 12G, en figure 12H, en figure 12I, en figure 12J, en figure 12K, et en figure 12L.

Dans ce mode de réalisation particulier, l'électrode 201 transparente et la reprise de contact sur le circuit électronique à travers le via 211 sont formés simultanément en déposant un même matériau transparent et conducteur.

En référence à la figure 12A, le procédé 300 débute avec l'étape S901, qui consiste à fournir le circuit électronique 22.

Ce circuit 22 comprend le plot de connexion 205 et la source de courant 204 commandée en tension. La source de courant 204 comprend l'électrode de commande 2042 (non représentée en figure 12A) reliée électriquement au plot de connexion 205 via la piste électrique 206. Le circuit 22 comprend en outre le circuit 207 électronique de pilotage.

L'étape S902, illustrée en figure 12B, est accomplie après l'étape S901 et consiste à former, sur le circuit électronique 22, l'élément électroluminescent 202.

Dans le cas d'un élément électroluminescent de type OLED, cette étape S902 comprend les sous-étapes successives suivantes :
- Formation de l'anode 2023 de l'élément électroluminescent 202,
- Formation de la couche 2022 organique,
- Formation de la cathode 2023.

Ces sous-étapes sont réalisées par une succession d'étapes de dépôt, de photolithographie et de gravure

L'étape S903, illustrée en figure 12C, suit l'étape S902 et consiste à encapsuler l'élément électroluminescent 202 par la couche protectrice transparente 203 (ou couche d'encapsulation). Cette couche protectrice 203 recouvre l'élément 202 électroluminescent et le plot de connexion 205.

Les étapes S904, S905, S906 et S907 qui suivent l'étape S903 visent à préparer la formation du via 211 conducteur en formant une ouverture 210 au niveau d'une zone adjacente à la zone 2024 (cf. figure 12F) de l'élément électroluminescent, cette ouverture 210 s'étendant depuis la surface 2031 de la couche protectrice 203 jusqu'au plot de connexion 205.

L'étape S904, illustrée en figure 12D, est une étape de dépôt d'une première couche sacrificielle 208 sur la surface 2031 de la couche protectrice 203. La couche sacrificielle 208 est de préférence une couche de résine photosensible.

L'étape S905, illustrée en figure 12E, est une étape de formation d'une première ouverture 209 à travers la première couche sacrificielle 208 dans la zone adjacente à la zone 2024, typiquement par insolation et développement de la résine photosensible. L'ouverture 209 débouche sur la couche protectrice 203.

L'étape S906, illustrée en figure 12F consiste à former une deuxième ouverture 210 dans le prolongement de la première ouverture 209, jusqu'au circuit électronique 22, par gravure verticale de la couche de protection 203 à travers la première couche sacrificielle 208. Autrement dit, la première couche sacrificielle 208 fait office de masque de gravure.

L'étape S907, illustrée en figure 12G, consiste à retirer la première couche sacrificielle 208.

Les étapes S908, S909, S910, S911 et S912 visent à former l'électrode transparente 201 et le via conducteur 211. L'électrode transparente 201 et le via conducteur 211 sont avantageusement formés par dépôt, photolithographie et gravure d'une même couche de matériau transparent et conducteur 201a.

L'étape S908, illustrée en figure 12H, suit l'étape S907 et consiste à déposer le matériau 201a transparent et conducteur sur toute la surface 2031 de la couche protectrice 203 et dans la deuxième ouverture 210.

Les étapes S909, S910, S911 et S912 visent notamment à délimiter l'électrode transparente 201 pour qu'elle présente la forme et les dimensions voulues.

L'étape S909, illustrée en figure 12I, consiste à déposer une deuxième couche sacrificielle 212 (typiquement une couche de résine photosensible) sur la couche de matériau conducteur et transparent 201a déposé précédemment.

L'étape S910, illustrée en figure 12J, est une étape de retrait localisé de la deuxième couche sacrificielle 212 (typiquement par insolation et développement couche de de la résine photosensible). Le retrait est effectué dans une zone 2011 située autour de la zone de l'élément électroluminescent 202.

L'étape S911, illustrée en figure 12K, est une étape de gravure de la couche de matériau transparent et conducteur 201a à travers la deuxième couche sacrificielle 212 (la deuxième couche sacrificielle 212 fait office de masque de gravure).

L'étape S912, illustrée en figure 12L, est une étape de retrait de la partie restante de la deuxième couche sacrificielle 212. Sur la couche 2031 protectrice, le matériau conducteur et transparent 201a forme l'électrode transparente 201, et sur les parois de la deuxième ouverture 210, il forme le via conducteur 211.

A l'issue de l'étape S912, l'élément d'imagerie 20 est formé.

Le dispositif d'imagerie 1 peut être fabriqué selon un procédé de fabrication dérivé du procédé de fabrication de l'élément d'imagerie 20 qui vient d'être décrit.

## Revendications

1. Elément d'imagerie (20, 20S) destiné à l'analyse d'un échantillon (E_{CH}) et comprenant :
- une électrode (201) adaptée à recevoir l'échantillon (E_{CH}),
ledit élément d'imagerie (20, 20S) étant **caractérisé en ce qu'**il comprend:
- un élément électroluminescent (202), disposé en vis-à-vis de l'électrode (201), et séparé de l'électrode par une couche protectrice (203) isolante,
- une source de courant (204) commandée en tension, configurée pour alimenter l'élément électroluminescent (202) en courant et comprenant une électrode de commande (2042) électriquement reliée à l'électrode (201), de sorte que ledit élément électroluminescent (202) génère une onde lumineuse (O_{20S}) en réponse à une tension (Vin_{20S}) provenant de l'échantillon (E_{CH}).

2. Elément d'imagerie (20, 20S) selon la revendication 1, dans lequel l'élément électroluminescent (202) est disposé entre la couche protectrice (203) et un substrat support (200) transparent ou semi-transparent.

3. Elément d'imagerie (20, 20S) selon l'une des revendications 1 à 2, dans lequel l'électrode (201) et la couche de protection (203) sont transparentes ou semi-transparentes.

4. Elément d'imagerie (20, 20S) selon l'une des revendications 1 à 3, dans lequel la source (204) de courant comprend un transistor (2041) ayant :
- une électrode de grille (2042) constituant l'électrode de commande de la source de courant ;
- une électrode de source (2043) reliée à l'élément électroluminescent ; et
- une électrode de drain (2044) reliée à une borne d'alimentation.

5. Elément d'imagerie (20, 20S) selon la revendication 4, dans lequel le transistor (2041) est un transistor en couches minces aussi appelé TFT.

6. Elément d'imagerie (20, 20S) selon l'une des revendications 4 à 5, dans lequel les dimensions latérales de l'électrode (201) sont supérieures aux dimensions latérales du transistor (2041).

7. Elément d'imagerie (20, 20S) selon l'une des revendications 1 à 6, dans lequel l'élément électroluminescent (202) est une diode électroluminescente organique.

8. Elément d'imagerie (20, 20S) selon l'une des revendications 1 à 6, dans lequel l'élément électroluminescent (202) est une diode électroluminescente inorganique, de préférence une micro-diode électroluminescente inorganique.

9. Elément d'imagerie (20, 20S) selon l'une des revendications 1 à 8, comprenant en outre un circuit électronique (207) de pilotage configuré pour générer une tension de commande (Vc) de la source de courant (204), ledit circuit électronique de pilotage (207) reliant électriquement l'électrode de commande (2042) de la source de courant (204) et l'électrode (201).

10. Dispositif d'imagerie (1) comprenant une pluralité d'éléments d'imagerie (20, 20S) selon l'une des revendications 1 à 9.

11. Dispositif d'imagerie (1) selon la revendication 10, dans lequel les éléments d'imagerie (20, 20S) sont agencés de manière à présenter un premier pas (P1) de répétition dans une première direction et un deuxième pas (P2) de répétition dans une deuxième direction sécante à la première direction.

12. Système (3) d'imagerie d'un échantillon (E_{CH}) comprenant :
- un dispositif d'imagerie (1) selon l'une des revendications 10 à 11,
- un capteur d'images (4) disposé en regard dudit dispositif d'imagerie (1) et adapté à former au moins une image de l'onde lumineuse (O_{20S}) générée par le dispositif d'imagerie (1) sous l'effet de l'échantillon (E_{CH}),
- un processeur (5) adapté à traiter l'image formée par le photodétecteur (4).

13. Procédé (100) d'analyse d'un échantillon (E_{CH}) à l'aide du dispositif d'imagerie (1) selon les revendications 10 à 11, comprenant les étapes suivantes :
- Dépôt (S101) de l'échantillon (E_{CH}) sur le dispositif d'imagerie (1) de sorte que l'échantillon (E_{CH}) soit au contact d'au moins une des électrodes (201) dudit dispositif d'imagerie (1), chaque électrode (201) en contact appartenant à un élément d'imagerie (20) correspondant dudit dispositif d'imagerie (1),
- Détection optique (S102) de l'activité électrique de l'échantillon (E_{CH}), comprenant une étape de collection (S1021) d'une onde lumineuse (O_{20S}) générée par l'élément électroluminescent (202) de l'élément d'imagerie correspondant à l'électrode en contact, l'onde lumineuse (O_{20S}) étant générée en réponse à une tension (Vin_{20S}) provenant de l'échantillon (E_{CH}).

14. Procédé d'analyse (100) selon la revendication 13, dans lequel l'onde lumineuse générée (O_{20S}) est collectée par un capteur d'images, disposé en regard du dispositif d'imagerie (1), et dans lequel l'étape de collection (S1021) est suivie d'une étape d'acquisition (S1022), par le photodétecteur (4), d'une image représentative de l'onde lumineuse (O_{20S}).

15. Procédé d'analyse (100) selon la revendication 14, dans lequel l'étape d'acquisition (S1022) de l'image représentative de l'onde lumineuse (O_{20S}) est suivie d'une étape de détermination (S1023) de la tension provenant de l'échantillon (E_{CH}) à partir de l'image acquise par le photodétecteur (4).

16. Procédé (900) de fabrication d'un élément (20) d'imagerie destiné à l'analyse d'un échantillon (ECH), comprenant les étapes suivantes :
- Fourniture (S901) d'un circuit électronique (22) comprenant un plot de connexion (205) et une source de courant (204) commandée en tension, la source de courant (204) comprenant une électrode de commande (2042) reliée électriquement au plot de connexion (205),
- Formation (S902), sur le circuit électronique (22), d'un élément électroluminescent (202),
- Formation (S903) d'une couche protectrice (203) transparente, ou semi-transparente, et isolante recouvrant l'élément électroluminescent (202) et le plot de connexion (205),
- Formation (S904, S905, S906, S907, S908, S909, S910, S911, S912), sur la couche protectrice (203) et en vis-à-vis de l'élément électroluminescent (202), d'une électrode transparente ou semi-transparente (201) adaptée à recevoir l'échantillon (ECH),
- Formation (S904, S905, S906, S907, S908, S909, S910, S911, S912), à travers la couche protectrice (203), d'un via conducteur (211) s'étendant depuis l'électrode transparente ou semi-transparente (201) jusqu'au plot de connexion (205).

17. Procédé (900) de fabrication selon la revendication 16, dans lequel le via conducteur (211) et électrode transparente ou semi-transparente (201) sont formés d'un même matériau (201a) respectivement conducteur et transparent, ou conducteur et semi-transparent.

## Patentansprüche

1. Bildgebungselement (20, 20S) zur Analyse einer Probe (E_{CH}), umfassend:
- eine Elektrode (201), die zur Aufnahme der Probe (E_{CH}) ausgelegt ist,
wobei das Bildgebungselement (20, 20S) **dadurch gekennzeichnet ist, dass** es umfasst:
- ein elektrolumineszentes Element (202), das der Elektrode (201) gegenüberliegend angeordnet und von der Elektrode durch eine isolierende Schutzschicht (203) getrennt ist,
- eine spannungsgesteuerte Stromquelle (204), die so ausgelegt ist, dass sie das elektrolumineszente Element (202) mit Strom versorgt, und die eine Steuerelektrode (2042) umfasst, die elektrisch mit der Elektrode (201) verbunden ist, sodass das elektrolumineszente Element (202) als Reaktion auf eine von der Probe (E_{CH}) kommende Spannung (Vin_{20S}) eine Lichtwelle (O_{20S}) erzeugt.

2. Bildgebungselement (20, 20S) nach Anspruch 1, in dem das elektrolumineszente Element (202) zwischen der Schutzschicht (203) und einem transparenten oder halbtransparenten Trägersubstrat (200) angeordnet ist.

3. Bildgebungselement (20, 20S) nach einem der Ansprüche 1 bis 2, in dem die Elektrode (201) und die Schutzschicht (203) transparent oder halbtransparent sind.

4. Bildgebungselement (20, 20S) nach einem der Ansprüche 1 bis 3, in dem die Stromquelle (204) einen Transistor (2041) umfasst, der aufweist:
- eine Gate-Elektrode (2042), die die Steuerelektrode der Stromquelle bildet;
- eine mit dem elektrolumineszenten Element verbundene Source-Elektrode (2043); und
- eine Drain-Elektrode (2044), die mit einem Versorgungsanschluss verbunden ist.

5. Bildgebungselement (20, 20S) nach Anspruch 4, bei dem der Transistor (2041) ein Dünnschichttransistor ist, der auch als TFT bezeichnet wird.

6. Bildgebungselement (20, 20S) nach einem der Ansprüche 4 oder 5, bei dem die seitlichen Abmessungen der Elektrode (201) größer sind als die seitlichen Abmessungen des Transistors (2041).

7. Bildgebungselement (20, 20S) nach einem der Ansprüche 1 bis 6, bei dem das elektrolumineszente Element (202) eine organische Leuchtdiode ist.

8. Bildgebungselement (20, 20S) nach einem der Ansprüche 1 bis 6, bei dem das elektrolumineszente Element (202) eine anorganische Leuchtdiode, vorzugsweise eine anorganische Mikro-Leuchtdiode, ist.

9. Bildgebungselement (20, 20S) nach einem der Ansprüche 1 bis 8, das ferner eine elektronische Ansteuerschaltung (207) umfasst, die so gestaltet ist, dass sie eine Steuerspannung (Vc) für die Stromquelle (204) erzeugt, wobei die elektronische Ansteuerschaltung (207) die Ansteuerelektrode (2042) der Stromquelle (204) und die Elektrode (201) elektrisch verbindet.

10. Bildgebungsvorrichtung (1) mit einer Vielzahl von Bildgebungselementen (20, 20S) nach einem der Ansprüche 1 bis 9.

11. Bildgebungsvorrichtung (1) nach Anspruch 10, bei der die Bildgebungselemente (20, 20S) so angeordnet sind, dass sie einen ersten Wiederholungsabstand (P1) in einer ersten Richtung und einen zweiten Wiederholungsabstand (P2) in einer zweiten Richtung aufweisen, die die erste Richtung schneidet.

12. Bildgebungssystem (3) für eine Probe (E_{CH}), umfassend:
- eine Bildgebungsvorrichtung (1) gemäß einem der Ansprüche 10 bis 11,
- einen Bildsensor (4), der gegenüber der Bildgebungsvorrichtung (1) angeordnet ist und dazu ausgelegt ist, mindestens ein Bild der von der Bildgebungsvorrichtung (1) unter dem Einfluss der Probe (E_{CH}) erzeugten Lichtwelle (O_{20S}) zu erzeugen,
- einen Prozessor (5), der dazu ausgelegt ist, das vom Bildsensor (4) erzeugte Bild zu verarbeiten.

13. Verfahren (100) zur Analyse einer Probe (E_{CH}) mit Hilfe der Bildgebungsvorrichtung (1) gemäß den Ansprüchen 10 bis 11, das die folgenden Schritte umfasst:
- Aufbringen (S101) der Probe (E_{CH}) auf die Bildgebungsvorrichtung (1), so dass die Probe (E_{CH}) mit mindestens einer der Elektroden (201) der Bildgebungsvorrichtung (1) in Kontakt steht, wobei jede in Kontakt stehende Elektrode (201) zu einem entsprechenden Bildgebungselement (20) der Bildgebungsvorrichtung (1) gehört,
- Optische Erfassung (S102) der elektrischen Aktivität der Probe (E_{CH}), umfassend einen Schritt zum Sammeln (S1021) einer Lichtwelle (O_{20S}), die von dem elektrolumineszenten Element (202) des der in Kontakt stehenden Elektrode entsprechenden Bildgebungselements erzeugt wird, wobei die Lichtwelle (O_{20S}) als Reaktion auf eine von der Probe (E_{CH}) stammende Spannung (Vin_{20S}) erzeugt wird.

14. Analyseverfahren (100) nach Anspruch 13, bei dem die erzeugte Lichtwelle (O_{20S}) von einem Bildsensor erfasst wird, der gegenüber der Bildgebungsvorrichtung (1) angeordnet ist, und bei dem nach dem Sammelschritt (S1021) ein weiterer Schritt (S1022) folgt, bei dem der Bildsensor (4) ein für die Lichtwelle (O_{20S}) repräsentatives Bild erfasst.

15. Analyseverfahren (100) nach Anspruch 14, bei dem nach dem Erfassungsschritt (S1022) des für die Lichtwelle (O_{20S}) repräsentativen Bildes ein weiterer Schritt (S1023) folgt, bei dem die von der Probe (E_{CH}) stammende Spannung anhand des vom Bildsensor (4) erfassten Bildes bestimmt wird.

16. Verfahren (900) zur Herstellung eines Bildgebungselements (20) zur Analyse einer Probe (ECH), das die folgenden Schritte umfasst:
- Bereitstellen (S901) einer elektronischen Schaltung (22) mit einer Anschlussklemme (205) und einer spannungsgesteuerten Stromquelle (204), wobei die Stromquelle (204) eine Steuerelektrode (2042) umfasst, die elektrisch mit der Anschlussklemme (205) verbunden ist,
- Bilden (S902) eines elektrolumineszenten Elements (202) auf der elektronischen Schaltung (22),
- Bilden (S903) einer transparenten oder halbtransparenten, isolierenden Schutzschicht (203), die das elektrolumineszente Element (202) und die Anschlussklemme (205) bedeckt,
- Bilden (S904, S905, S906, S907, S908, S909, S910, S911, S912) auf der Schutzschicht (203) und gegenüber dem elektrolumineszenten Element (202) einer transparenten oder halbtransparenten Elektrode (201), die zur Aufnahme der Probe (ECH) geeignet ist,
- Bilden (S904, S905, S906, S907, S908, S909, S910, S911, S912) durch die Schutzschicht (203) hindurch einer leitfähigen Durchkontaktierung (211), die sich von der transparenten oder halbtransparenten Elektrode (201) bis zur Anschlussklemme (205) erstreckt.

17. Herstellungsverfahren (900) nach Anspruch 16, bei dem die leitfähige Durchkontaktierung (211) und die transparente oder halbtransparente Elektrode (201) aus demselben Material (201a) gebildet sind, das jeweils leitfähig und transparent bzw. leitfähig und halbtransparent ist.

## Claims

1. Imaging element (20, 20S) for analysis of a sample (E_{CH} and comprising:
- an electrode (201) adapted to receive the sample (E_{CH}),
said imaging element (20, 20S) being **characterized in that** it comprises:
- a light-emitting element (202), disposed opposite the electrode (201), and separated from the electrode by an insulating protective layer (203),
- a voltage-controlled current source (204) configured to supply current to the light-emitting element (202) and comprising a control electrode (2042) electrically connected to the electrode (201), so that said light-emitting element (202) generates a light wave (O_{20S}) in response to a voltage (Vin_{20S}) coming from the sample (E_{CH}).

2. Imaging element (20, 20S) according to claim 1, wherein the light-emitting element (202) is disposed between the protective layer (203) and a transparent or semi-transparent support substrate (200).

3. Imaging element (20, 20S) according to one of claims 1 to 2, wherein the electrode (201) and the protective layer (203) are transparent or semi-transparent.

4. Imaging element (20, 20S) according to one of claims 1 to 3, wherein the current source (204) comprises a transistor (2041) having:
- a gate electrode (2042) constituting the control electrode of the current source;
- a source electrode (2043) connected to the light-emitting element; and
- a drain electrode (2044) connected to a power supply terminal.

5. Imaging element (20, 20S) according to claim 4, wherein the transistor (2041) is a thin film transistor also known as a TFT.

6. Imaging element (20, 20S) according to one of claims 4 to 5, wherein the lateral dimensions of the electrode (201) are greater than the lateral dimensions of the transistor (2041).

7. Imaging element (20, 20S) according to one of claims 1 to 6, wherein the light-emitting element (202) is an organic light-emitting diode.

8. Imaging element (20, 20S) according to one of claims 1 to 6, wherein the light-emitting element (202) is an inorganic light-emitting diode, preferably an inorganic micro light-emitting diode.

9. Imaging element (20, 20S) according to one of claims 1 to 8, further comprising an electronic driver circuit (207) configured to generate a control voltage (Vc) of the current source (204), said electronic driver circuit (207) electrically connecting the control electrode (2042) of the current source (204) and the electrode (201).

10. Imaging device (1) comprising a plurality of imaging elements (20, 20S) according to one of claims 1 to 9.

11. Imaging device (1) according to claim 10, wherein the imaging elements (20, 20S) are arranged so as to have a first pitch (P1) in a first direction and a second pitch (P2) in a second direction intersecting the first direction.

12. System (3) for imaging a sample (E_{CH} comprising:
- an imaging device (1) according to one of claims 10 to 11,
- an image sensor (4) disposed opposite said imaging device (1) and adapted to form at least one image of the light wave (O_{20S}) generated by the imaging device (1) under the effect of the sample (E_{CH}),
- a processor (5) adapted to process the image formed by the photodetector (4).

13. Method (100) for analysing a sample (E_{CH} using the imaging device (1) according to claims 10 to 11, comprising the following steps of:
- Depositing (S101) the sample (E_{CH}) onto the imaging device (1) so that the sample (E_{CH}) is in contact with at least one of the electrodes (201) of said imaging device (1), each electrode (201) in contact belonging to a corresponding imaging element (20) of said imaging device (1),
- Optically detecting (S102) the electrical activity of the sample (E_{CH}), comprising a step of collecting (S1021) a light wave (O_{20S}) generated by the light-emitting element (202) of the imaging element corresponding to the electrode in contact, the light wave (O_{20S}) being generated in response to a voltage (Vin_{20S}) coming from the sample (E_{CH}).

14. Analysis method (100) according to claim 13, wherein the light wave (O_{20S}) generated is collected by an image sensor disposed opposite the imaging device (1), and wherein the collection step (S1021) is followed by a step (S1022) of acquiring, by the photodetector (4), an image representative of the light wave (O_{20S}).

15. Analysis method (100) according to claim 14, wherein the step (S1022) of acquiring the image representative of the light wave (O_{20S}) is followed by a step (S1023) of determining the voltage coming from the sample (E_{CH}) from the image acquired by the photodetector (4).

16. Method (900) for manufacturing an imaging element (20) for analysis of a sample (E_{CH}), comprising the following steps of:
- Providing (S901) an electronic circuit (22) comprising a bonding pad (205) and a voltage-controlled current source (204), the current source (204) comprising a control electrode (2042) electrically connected to the bonding pad (205),
- Forming (S902), on the electronic circuit (22), a light-emitting element (202),
- Forming (S903) a transparent or semi-transparent insulating protective layer (203) covering the light-emitting element (202) and the bonding pad (205),
- Forming (S904, S905, S906, S907, S908, S909, S910, S911, S912), on the protective layer (203) and opposite the light-emitting element (202), a transparent or semi-transparent electrode (201) adapted to receive the sample (E_{CH}),
- Forming (S904, S905, S906, S907, S908, S909, S910, S911, S912), through the protective layer (203), a conductive via (211) extending from the transparent or semi-transparent electrode (201) to the bonding pad (205).

17. Manufacturing method (900) according to claim 16, wherein the conductive via (211) and the transparent or semi-transparent electrode (201) are formed from a same material (201a) which is conductive and transparent, or conductive and semi-transparent respectively.
